(19) **Europäisches Patentamt**

**European Patent Office**

**Office européen des brevets**

(11) **EP 1 058 116 A2**

(12) **EUROPÄISCHE PATENTANMELDUNG**

| | |
|---|---|
| (43) Veröffentlichungstag:<br>**06.12.2000 Patentblatt 2000/49** | (51) Int. Cl.[7]: **G01N 33/68**, G01N 33/543,<br>G01N 33/60, G01N 33/569 |

(21) Anmeldenummer: **00110703.6**

(22) Anmeldetag: **19.05.2000**

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **02.06.1999 DE 19925405**

(71) Anmelder:
• **Bieger, Wilfried P., Priv. Doz. Dr. med. habil.**
**80335 München (DE)**
• **Von Baehr, Volker, Dr. med.**
**10179 Berlin (DE)**

(72) Erfinder:
• **Von Baehr, Volker, Dr.med.**
**10179 Berlin (DE)**
• **Bieger, Wilfried P., Priv. Doz.Dr.med.**
**86199 Augsburg (DE)**
• **Volk, Hans Dieter, Prof. Dr.med.**
**10178 Berlin (DE)**

(74) Vertreter:
**Schwarzensteiner, Marie-Luise et al**
**Grape & Schwarzensteiner**
**Patentanwälte**
**Sebastiansplatz 7**
**80331 München (DE)**

(54) **Verfahren zum Nachweis von spezifisch antigenreaktiven Lymphozyten sowie ein Nachweis-Kit zur Durchführung dieses Verfahrens**

(57) Die Erfindung betrifft ein Verfahren zum in vitro-Nachweis von spezifisch antigen- bzw. allergenreaktiven Lymphozyten, bei dem man einer auf ein Antigen bzw. Allergen zu untersuchenden Zellkultur wenigstens eine Substanz zugibt, welche eine unspezifische Zell-Proliferation unterdrückt.

**EP 1 058 116 A2**

**Beschreibung**

Allgemein betrifft die vorliegende Erfindung ein Verfahren zum Nachweis bzw. zur Diagnose von durch spezifische Allergene bzw. Antigene hervorgerufenen Erkrankungen, von Autoimmunerkrankungen hervorrufenden T-Zellen oder von ausgeprägten T-zellulären Sensibilisierungen, welche latente Infektionen mit intrazellulär persistierenden Erregern belegen.

Ein wohlbekanntes in vivo-Verfahren zum Nachweis spezifischer Sensibilisierungen gegenüber sogenannten Allergenen sind Hautteste. Bei diesen Tests werden verschiedene Allergene beispielsweise auf die Rückenhaut einer zu untersuchenden Person aufgetragen. Nach etwa 48 bis 72 Stunden wird eine visuelle Bewertung des Tests vorgenommen. Positiv wird ein Allergen dann bewertet, wenn an seiner Auftragsstelle Rätung und Induration aufgetreten sind. Beim Auftreten einer oder mehrerer dieser Erscheinungen ist es wahrscheinlich, daß die untersuchte Person gegen das bzw. die Allergene sensibilisiert ist, d.h. über spezifische Gedächtniszellen verfügt.

Ein Vorteil dieses Nachweisverfahrens ist es, daß es beispielsweise unmittelbar von einem Arzt in der Praxis durchgeführt und ausgewertet werden kann und relativ kostengünstig ist.

Dieses Verfahren beinhaltet jedoch auch verschiedene Nachteile, indem das Ergebnis einer subjektiven Bewertung einer den Test durchführenden Person unterliegt, unspezifische Hautirritationen häufig falsch-positive Reaktionen auslösen, der zu untersuchende Patient durch den Nachweis möglicherweise erst sensibilisiert wird, das Testergebnis durch beispielsweise den Hauttyp des Patienten beeinflußt werden kann und eine Standardisierung zur Bewertung des Ergebnisses nicht zuletzt dadurch nicht möglich ist.

Ein Verfahren, welches die vorangehend beschriebenen Schwierigkeiten hinsichtlich der Zuverlässigkeit eines erhaltenen Testergebnisses im wesentlichen ausschließt, ist ein in-vitro-Verfahren, das ursprünglich als Funktionstest des zellulären Immunsystems eingeführt worden ist und als solcher auch heute noch überwiegend in der Klinik Anwendung findet, der sogenannte Lymphozytentransformationstest (LTT).

Bei diesem Test werden mononukleäre Zellen eines Patienten unter Zugabe der zu testenden Substanzen in Zellkulturplatten für mehrere Tage inkubiert. Dazu gewinnt man zunächst eine mononukleäre Zellfraktion, welche Lymphozyten und Monozyten enthält, aus peripherem bzw. venösem Blut einer zu untersuchenden Testperson und inkubiert diese mit möglichen Allergenen bzw. Antigenen. Sind in der Zellfraktion antigen- bzw. allergenreaktive Lymphozyten (T-Zellen, B-Zellen) vorhanden, werden diese durch den Kontakt aktiviert, d.h. zur Proliferation stimuliert. Die Aktivierung bzw. Proliferation wird in der Routinelabordiagnostik derzeit durch Messung der Einbaurate eines radioaktiv-markierten DNS-Bausteins, insbesondere von $^3$H-Thymidin, nachgewiesen und quantifiziert.

Die Vorteile dieser in-vitro-Methode gegenüber dem vorher erläuterten Epikutantest sind eine Objektivität der Auswertung des Testes, keine Gefährdung des Patienten durch eine möglicherweise erst eintretende Sensibilisierung, eine Quantifizier- und Standardisierbarkeit des Testes und die Unabhängigkeit der Testaussage vom Hauttyp des Patienten.

Jedoch weist auch dieses Testverfahren, trotz der eben genannten Vorteile, Nachteile bei der Durchführung auf, welche insbesondere zu falsch-positiven oder auch falsch-negativen Ergebnissen führen können, welche insbesondere für die nachfolgende Behandlung und/oder Medikamentierung eines Patienten von erheblichem Nachteil sein können.

Ein solches Problem ist das Auftreten unspezifischer Reaktionen. Bei dem herkömmlichen LTT-Test ist es neben einer längeren Inkubationszeit erforderlich, größere Zellmengen einzusetzen, um eine eventuell geringe Anzahl antigen- bzw. allergenspezifischer Lymphozyten, d.h. Gedächtniszellen, im Blut unter Antigen- bzw. Allergenstimulation zur Proliferation zu bringen, damit ein hinreichend signifikantes Meßsignal durch zellgebundene Radioaktivität erreicht wird. Derartige Ansätze erfordern mindestens 1 Million Zellen in 1 ml Kulturmedium.

Ein zweites Problem ist die Unsicherheit der Testaussage aufgrund einer möglicherweise auftretenden, stark erhöhten Basalproliferation, die besonders bei Nutzung von autologem Serum in ca. 30 - 40 % der Fälle auftritt. Beim herkömmlichen LTT wird die unter Antigen- bzw. Allergenzufuhr erzielte radioaktive Einbaurate zu der ohne Antigen- bzw. Allergenzufuhr gemessenen radioaktiven Einbaurate, welche den Basalwert angibt, ins Verhältnis gesetzt. Dieser Wert, welcher als Stimulationsindex (SI) bezeichnet wird, ist der Quotient aus antigen- bzw. allergeninduzierter Proliferation und eben dieser Basalproliferation. Bei einer stark erhöhten Basalproliferation, zum Beispiel durch eine Präaktivierung der Zellen bei einer vorliegenden Infektion, bei bestimmten konstitutionellen Bedingungen einiger Patienten, bei hoher Zellzahl im Ansatz, kann der SI-Wert auch bei einer positiven Reaktion nur Werte erreichen, mit welchen eine Aussage gegebenenfalls nur als fraglich bezeichnet werden kann.

Einen weiteren Nachteil stellt die Tatsache dar, daß der reagierende Effektorzelltyp nicht bestimmt und damit keine Aussage über dessen klinische Relevanz getroffen werden kann. Ein hoher Stimulationsindex bedeutet, daß im antigen- bzw. allergenstimulierten Ansatz deutlich mehr Zellen aktiviert werden als im Basalansatz. Eine Aussage, ob es sich dabei um Zellen mit zytotoxischem Effektorpotential (TNF-α, IFN-γ), oder um Toleranz induzierende Zellen (IL4, IL10) handelt, kann nicht gemacht werden. Über die klinische Relevanz der Sensibilisierung und deren Aktivität kann daher keine Aussage getroffen werde. Nachgewiesen werden kann lediglich das Vorhandensein spezifischer Gedächtniszellen.

Aufgabe der vorliegenden Erfindung ist es daher, die dem Stand der Technik gemeinsamen Probleme bzw. Nachteile zu überwinden. Insbesondere sollte es möglich gemacht werden, einen zuverlässig positiven Test auf möglicherweise vorliegende Sensibilisierungen, Autoimmunerkrankungen auslösende Zellen oder Erkrankungen, welche durch eine latente Infektion hervorgerufen werden bzw. worden sind, zur Verfügung zu stellen.

Idee der vorliegenden Erfindung war es daher, eine Möglichkeit zu finden, mit welcher eine unspezifische Zell-Proliferation bzw. eine nicht antigen- bzw. allergenspezifische Reaktion unterdrückt bzw. sogar unterbunden werden kann.

Überraschenderweise konnte diese Aufgabe durch ein Verfahren zum in-vitro-Nachweis von spezifisch antigen- bzw. allergenreaktiven Lymphozyten gelöst werden, bei dem man einer auf ein Antigen bzw. Allergen zu untersuchenden Zellkultur wenigstens eine Substanz zugibt, welche eine unspezifische Zell-Proliferation unterdrückt.

Vorzugsweise wird diese Substanz bzw. diese Substanzen aus der Familie der Human-Interferone ausgewählt. Als besonders geeignet hat sich im Versuch der Einsatz von $\alpha$-Interferon ($\alpha$-IFN), insbesondere von rekombinantem $\alpha$-Interferon gezeigt.

Durch den Einsatz dieser Substanz(en) in einem herkömmlichen Lymphozytentransformationstest, der hier lediglich beispielhaft zur Erläuterung der Erfindung angegeben wird, wird die unspezifische Aktivierung und Proliferation von T-Lymphozyten, d.h. Immunzellen, welche in der Zellkultur vorliegen können, gehemmt oder unterdrückt, während überraschenderweise ein spezifisches Triggern, d.h. Aktivieren und Proliferieren von antigen- bzw. allergenspezifischen Lymphozyten bzw. Gedächtniszellen, verstärkt oder nicht beeinflußt wird. Hierdurch wird die basale, spontane Proliferation der Immunzellen im Test herabgesetzt und somit nimmt der Stimulationsindex (SI), d.h. der Quotient aus spezifischer Proliferation und Basalproliferation im Falle einer spezifischen Sensibilisierung eindeutig positive Werte an. Es hat sich gezeigt, daß das Signalabstandsverhältnis bei der Zellzahlbestimmung durch Messung der eingebauten Radioaktivität (z.B. ³H-Thymidin) zwischen antigen- bzw. allergenspezifisch stimulierten Zellen und Basalproliferation ohne Antigen- bzw. Allergenzufuhr um den Faktor 2 bis 3 gesteigert werden kann, was falsch- oder fraglichpositive Ergebnisse bei dem erfindungsgemäßen Test ausschließt. Erfindungsgemäß wird ein Stimulationsindex von größer als 3 als positiv angesehen, Werte zwischen 2 und 3 als fraglich positiv und darunter als negativ.

Ein weiterer und wesentlicher Vorteil des erfindungsgemäßen Verfahrens ist, daß die vorstehend erläuterte Steigerung des Signalabstandsverhältnisses die Verwendung geringerer Gesamt-Zellmengen im Ansatz als herkömmlich erlaubt. So ist ein Zellansatz in Mikrokulturen von beispielsweise 250.000 Zellen in 200 µl Kulturmedium möglich. Die Untersuchungen in diesem Zusammenhang haben gezeigt, daß die Menge eingesetzter Zellen nicht nur reduziert werden kann, wodurch die Durchführung des Testes auf beispielsweise 96-well-Mikrotiterplatten möglich wird, sondern mit dieser geringen Zellmenge signifikant bessere Ergebnisse erzielt werden.

Den wichtigsten Fortschritt des erfindungsgemäßen Verfahrens stellt der Umstand dar, daß es damit möglich ist, im Lymphozytentransformationstest zum Nachweis von Sensibilisierungen gegen Allergene, Infektionsantigene und Autoantigene neben den Immunzellen auch das Serum des betreffenden Patienten einzusetzen. Dieses autologe Serum kann Antikörper enthalten, die zu einer Verbesserung der Antigenpräsentation und damit zu einer effektiven Stimulation der T-Lymphozyten des Patienten führen. Mit autologem Serum kann das spezifische immunologische Milieu des individuellen Patienten im Testansatz erhalten werden, was zu einer Verdopplung bis Verdreifachung der Stimulationsindizes führt. Ohne Anwendung des erfindungsgemäßen Verfahrens ist die Verwendung autologen Serums im Lymphozytentransformationstest kaum oder nicht möglich, da es dabei in ca. 30 - 50 % der Fälle zu einer drastischen Erhöhung der Basalproliferation kommt, was die Bemessung der spezifischen Stimulation durch Allergene/Antigene äußerst erschwert. Es war deshalb notwendig, durch Vorhertestung geeignete Humanseren mit geringer oder keiner Beeinflussung der Testzellen auszuwählen. Die Notwendigkeit dieser kostenaufwendigen Prozedur entfällt bei der Anwendung des erfindungsgemäßen Verfahrens.

Bei dem erfindungsgemäßen Verfahren kann eine aus venösem Blut gewonnene mononukleäre, Lymphozyten und Monozyten enthaltende, Zellfraktion eingesetzt werden, so daß eine Abreicherung der letzteren, wie im MELISA-Test, welcher in der EP 0 558 566 B1 beschrieben ist, nicht mehr erforderlich ist. Damit ist eine einfache Aufbereitung der Zellkultur möglich, die eine weitgehende Standardisierung des Testes zuläßt.

Erfindungsgemäß stellt man die zur Durchführung des Verfahrens zu verwendende Zellkultur auf eine Zellzahl von etwa 1,0 bis 1,5 x 10⁶ Zellen/ml ein. In diesem Bereich werden signifikant aussagekräftige Ergebnisse erhalten.

Gute Ergebnisse werden erhalten, wenn man der so gewonnenen Zellsuspension die Substanz(en), welche eine unspezifische Zellproliferation unterdrückt bzw. unterdrücken, bevorzugt humanes $\alpha$-Interferon (h-$\alpha$-IFN) in einer Menge zugibt, daß sie in der Zellkultur in einer Endkonzentration von etwa 100 bis 140 IU/ml vorliegt bzw. vorliegen.

Erfindungsgemäß wird diese so eingestellte Zellkultur in üblicher Weise unter in-vitro-Bedingungen inkubiert. Ein genaues Beispiel wird im Anschluß an diese allgemeine Beschreibung angegeben werden.

Das erfindungsgemäße Verfahren zeigt den Vorteil, daß durch die Hemmung der unspezifischen Aktivierung durch beispielsweise das $\alpha$-Interferon, neben der Proliferation weitere mit der Aktivierung vorhandene Ereignisse erfaßt werden können, indem es möglich ist, die in den Kulturüberstand sezernierten Zytokine zu bestimmen, da deren Muster durch den Zusatz von z.B. α-IFN nicht verändert wird. Daraus läßt sich nicht nur der sezernierte Zelltyp, sondern auch die klinische Relevanz der bestehenden Sensibilisierung bestimmen.

Erfindungsgemäß gewinnt man daher nach beendeter Inkubationsphase die Kulturüberstände zellfrei und bestimmt in üblicher Weise die darin enthaltenen Zytokine oder man inkubiert die Zellsuspension im weiteren mit einem radioaktiven DNS-Baustein, vorzugsweise $^3$H-Thymidin, der sich in die proliferierenden Zellen einbaut, welche dann geerntet und analysiert werden.

In geeigneter Weise kann man diesen Test in einer vollautomatisierten Analyseapparatur durchführen. Jedoch wird es erfindungsgemäß vorgezogen, daß man den Test auf sogenannten Mikrotiterplatten, insbesondere 96-well Mikrotiterplatten durchführt. Aufgrund der sehr geringen zu verwendenden Mengen an Zellen (Lymphozyten/Monozyten-Fraktion), Antigenen bzw. Allergenen sowie radioaktivem DNS-Baustein können solche Platten in einem Durchlauf neben den verdächtigen Antigenen bzw. Allergenen, Kontrollen zur Untersuchung der Zellviabilität und der proliferativen Aktivität, wie PWM oder Tetanus, und Basalansätze, d.h. solche, ohne Antigen bzw. Allergen, mit sich führen. Damit verringern sich die Testdauer und die Testkosten wesentlich.

Für Routineuntersuchungen ist es besonders günstig und deshalb bevorzugt, Mikrotiterplatten zu verwenden, welche in einer Weise mit Antigenen bzw. Allergenen belegt sind, daß diese über einen längeren Zeitraum lagerbar sind und so zur Standardisierung des Verfahrens beitragen.

Für routinemäßige Untersuchungen empfiehlt es sich, daß man pro zu testendem Antigen bzw. Allergen zur Ermittlung eines Mittelwertes und damit zur Erhöhung der Genauigkeit des Verfahrens drei Ansätze fährt, man gleichzeitig die Basalproliferation, d.h. ohne Zugabe von Antigen bzw. Allergen, bestimmt und damit die erhaltenen Ergebnisse ins Verhältnis setzt. Der Stimulationsindex (SI) wird nach der Gleichung

$$SI = cpm \text{ in induziert proliferierender Kultur}/cpm \text{ in basal proliferierender Kultur}$$

berechnet. Je höher dieser Wert ist, desto sicherer ist die Aussagekraft des Tests im Falle einer positiven Reaktion.

Das erfindungsgemäße Verfahren eignet sich daher besonders gut zum in-vitro-Nachweis von Sensibilisierungen, d.h. Allergien, insbesondere auf Schwermetalle, wie insbesondere Palladium-, Nickel- und/oder Goldallergien. Selbstverständlich können damit auch Allergien gegen alle andere Metalle, z.B. Quecksilber, Silber, Cadmium, Chrom, Platin, Titan, Zinn oder auch die Amalgamlegierung nachgewiesen werden. Beispiele hierfür sind im folgenden angegeben.

Das erfindungsgemäße Verfahren, kann auch erfolgreich zum Nachweis autoreaktiver T-Zellen eingesetzt werden, also Zellen, die sich gegen körpereigene Zellstrukturen richten. Das ist beispielswiese bei Multipler Sklerose der Fall. Ein Beispiel hierfür ist im folgenden angegeben.

Das erfindungsgemäße Verfahren ist auch zum Nachweis intrazellulär persistierender Infektionen, z.B. Chlamydien, Borrelien, Tuberkulose, geeignet, welche sich in verschiedenen Krankheitsbildern ausdrücken können. Ein Beispiel wird nachfolgend angegeben.

Gegenstand der Erfindung ist auch ein Nachweis-Kit zur Durchführung des Verfahrens, welcher wenigstens die mit Antigen bzw. Allergen und Kontrollsubstanzen beladenen Mikrotiterplatten umfaßt.

Im folgenden werden zur Erläuterung des erfindungsgemäßen Verfahrens Einzelheiten der Durchführung des Tests beispielhaft angegeben.

Zunächst werden Kulturplatten zum Inkubieren der Patientenproben vorbereitet. Verwendet werden vorzugsweise 96-well Mikrotiterzellkulturplatten mit flachem Boden (z.B. Cellstar, Greiner). Aus Gründen der Praktikabilität sowie der Vergleichbarkeit zwischen Patienten werden die mit Antigen bzw. Allergen belegten Platten in größeren Mengen vorgefertigt und bei -20°C bis zu ihrem Gebrauch gelagert. Diese Platten, welche in Verbindung mit einem Nachweis-Kit ebenfalls Gegenstand der vorliegenden Erfindung sind, können über einen Zeitraum von wenigstens 2 Monaten unter diesen Bedingungen aufbewahrt werden. Zur Belegung der Platten werden etwa 20 μl einer geeignet konzentrierten Antigenverdünnung in sterilem Phosphatpuffer (PBS) mittels eines automatisierten Pipettiergerätes (Stratec, Vivace) belegt. Selbstverständlich sollte diese Mengenangabe nur als Beispiel dienen, den Umfang der Erfindung jedoch nicht beschränken.

Maximal 8 Stunden vor der Verarbeitung werden einem zu untersuchenden Patienten 20 bis 30 ml venöses Blut entnommen. Hierzu können heparinisierte oder herkömmliche Blutabnahmemonovetten mit Citrat oder EDTA verwendet werden. Sind längere Lagerzeiten vor der Verarbeitung, beispielsweise aufgrund von langen Transportwegen der Proben vom Arzt zum Untersuchungslabor, erforderlich, verwendet man spezielle CPT-Röhrchen (Becton Dickenson), in denen direkt nach der Blutabnahme durch Zentrifugieren entsprechend den Herstellerangaben eine Abtrennung der mononukleären Zellfraktion erfolgt.

Die Abtrennung der mononukleären Zellen, d.h. Lymphozyten und Monozyten, von Granulozyten, Thrombozyten und Erythrozyten erfolgt mittels Dichtegradientenzentrifugation über Ficoll Paque. 20 bis 30 ml Vollblut werden im Verhältnis 1 : 2 (Volumen/Volumen) mit Phosphatpufferlösung verdünnt. Zweimal 50 ml davon pro Patient in Trennröhrchen (Accuspin, Sigma) gebracht und anschließend durch Gradientenzentrifugation bei 800 g 20 Minuten lang aufgetrennt. Dann wird die gut sichtbare helle Zwischenphase, welche hauptsächlich aus Lymphozyten und Monozyten besteht, abgenommen und in ein steriles 50 ml Röhrchen (Saarstedt) überführt und mit Phosphatpufferlösung (PBS) zweimal gewaschen und jeweils nach dem Waschen bei 250 g 10 Minuten lang zentrifugiert.

In ähnlicher Weise verfährt man bei der Gewinnung der mononukleären Zellfraktion aus den CPT-Röhrchen. Hier kann jedoch der Überstand nach kräftigem Schütteln der Abnahmemonovetten sofort durch Abkippen in ein 50 ml Röhrchen (Saarstedt) übergeführt werden. Nach dem Auffüllen des Röhrchens mit PBS erfolgen ebenfalls zwei Waschschritte.

Das jeweils aus dem letzten Waschschritt resultierende Zellpellet wird anschließend im Kulturmedium resuspendiert und auf eine Zellzahl von 1,0 bis 1,5 x $10^6$ Zellen/ml eingestellt. Das im Rahmen der vorliegenden Erfindung verwendete Kulturmedium ist RPMI 1640, welches 5 % humanes AB-Poolplasma (Sigma, Deisenhofen, BRD, oder vorzugsweise 5 - 10 % autologes Serum) sowie Glutamin (4 mM, Sigma), Gentamycin (30 μg/ml, Biochrom) und HEPES (0,02 M, Sigma) enthält. Die Zellzahlbestimmung erfolgt vorzugsweise mit einem Coulter-Zellzählgerät.

Diesen Zellsuspensionen wird erfindungsgemäß rekombinantes, humanes α-Interferon (z.B. von Biosource, Camarillo, USA, Kat.-Nr. PHC 4814 oder anderen Herstellern) zugegeben. Dazu wird es in serumfreiem Kulturmedium vorverdünnt und anschließend zur Zellsuspension gegeben. Im Kulturansatz liegt das α-Interferon in einer Konzentration von 125 U/ml vor.

Das Ansetzen der Kultur erfolgt auf den bereits beschriebenen mit Antigen bzw. Allergen beladenen 96-well-Mikrotiterplatten in drei Ansätzen pro zu testender Substanz. Dabei werden 200 μl der bereiteten Zellsuspension in jedes well gebracht. Die Platten werden dann in einem Inkubator bei 37°C mit 5 % $CO_2$ in Luftatmosphäre 5 ½ Tage unter sterilen Bedingungen inkubiert.

Die basale Proliferation wird ebenfalls in drei wells bestimmt, denen anstelle der Antigenverdünnung reine Phosphatpufferlösung zugegeben wurde. Zur Einschätzung der Zellviabilität und der proliferativen Kapazität der verwendeten Zellen dienen als Positivkontrollen PWM (Mitogen) sowie Tetanus (Recall-Antigen).

Am Abend des fünften Tages werden 0,25 μCi Methyl-$^3$H-Thymidin (Amersham, U.K.) zu den 200 μl Zellsuspension in jedem well gegeben. Die Endkonzentration beträgt 1,25 μCi/ml. Danach werden die Zellen für weitere 15 Stunden bei 37°C mit 5 % $CO_2$ in Luftatmosphäre inkubiert.

Nach Ablauf der zweiten Inkubationsphase über Nacht werden die Zellen in einem automatisierten Zellerntegerät (Cell Harvester, Inotech) geerntet. Zum Waschen wird destilliertes Wasser verwendet. Während dieses Ernteschrittes werden die Zellen separat für jedes der 96 wells in einen Glasphaserfilter gezogen, der anschließend auf den Gehalt an Radioaktivität pro well untersucht werden kann. Die Messung der Radioaktivität in cpm (counts per minute) erfolgt mittels eines automatisierten Meßsystems (β-Counter, Wallac, Finnland). Nach Berechnung der Mittelwerte aus den jeweiligen Dreifachansätzen erfolgt die Berechnung des Stimulationsindexes SI nach der vorher angegebenen Gleichung.

Ein SI-Wert von größer 3 wird als positiv angesehen, ein SI zwischen 2 und 3 als fraglich positiv, darunter ist das Ergebnis negativ.

Parallel zu den radioaktiv zu markierenden Kulturansätzen werden identische Ansätze mitgeführt, denen kein radioaktives Material zugesetzt wird, um sezernierte Zytokine zu bestimmen. Am fünften Tag werden die Kulturüberstände zellfrei aus den wells gewonnen und in ihnen die Konzentration von z.B. γ-IFN, IL-4, IL-10, TNF-α, IL-2 und GM-CSF mittels kommerziell erhältlichen ELISA-Testbestecken nach Angaben der Hersteller bestimmt. Ähnlich der Proliferationsmessung werden die gemessenen Zytokinspiegel zu den in Kontrollkulturen ermittelten Werten ins Verhältnis gesetzt.

Die Hemmung der unspezifischen Aktivierung durch das α-Interferon ermöglicht es so, neben der Proliferation weitere aktivierungsassoziierte Ereignisse im Testansatz zu erfassen. Das sind die Analyse phänotypischer Eigenschaften der Zellen, d.h. morphologische Aktivierung, Oberflächenexpression von Aktivierungsmarkern, die Quantifizierung der allergen- bzw. antigeninduzierten Zytokinsekretion durch Messung der sezernierten Zytokine im Zellüberstand sowie die Analyse funktioneller Eigenschaften der Zellen, beispielsweise im Zytotoxizitätstest.

Die nachfolgenden Ausführungsbeispiele zeigen die möglichen Anwendungsbereiche des erfindungsgemäßen Verfahrens und seine Zuverlässigkeit. Die Ergebnisse sind in den Tafeln 1 bis 5 zusammengefaßt.

Beispiel 1

Bei diesem Beispiel wird eine zellvermittelte Hypersensivitätsreaktion gegenüber Palladium im Fall einer Patientin mit nachgewiesener Palladium-Langzeitexposition durch Bauchnabelpiercing mit einem palladiumhaltigen Ring gezeigt. Die Patientin zeigte leichte Erschöpfbarkeit, Kopfschmerz, Infektanfälligkeit, jedoch keine Lokalreaktion.

Einzelheiten des Testergebnisses zeigt die Tafel 1. Die durch Palladium induzierte zelluläre Aktivierung wurde durch Analyse der Zellproliferation und Ermittlung des Stimulationsindex, wie er vorher definiert wurde, nachgewiesen. Der SI-Wert ist eindeutig positiv. Der Nachweis konnte auch durch γ-IFN-Sekretion in den Zellüberstand unter der Wirkung von 1,5 µg/ml bzw. 3,0 µg/ml Palladium geführt werden. Unter der Wirkung von 3 µg/m Palladium erfolgte als Zeichen der T-Zellen-Aktivierung ein Anstieg der HLA-DR-Expression auf CD4 positiven und CD8 positiven Lymphozyten.

Beispiel 2

Die untersuchte Patientin zeigte einen erheblich reduzierten Allgemeinzustand, Gleichgewichts- und Sensibilitätsstörungen, Kopfschmerzen, Wortfindungsschwierigkeiten sowie eine deutliche Voralterung. Es bestand dringender klinischer Verdacht auf Multiple Sklerose, ein Krankheitsbild, bei dem autoreaktive T-Zellen gegen Nervengewebe (z.B. MOG myelinbasisches Protein) verantwortlich gemacht werden.
Einzelheiten des Testergebnisses zeigt die Tafel 2. Es konnten autoreaktive T-Zellen gegenüber myelinassoziierten Peptidsegmenten durch Bestimmung des SI-Wertes, welcher deutlich über 3 lag und durch IFN-γ-Sekretion in den Kulturüberstand nachgewiesen werden.

Beispiel 3

Bei diesem Beispiel wird eine zellvermittelte Hypersensivität gegenüber Nickel durch Nickel-induzierte Sekretion von TNF-α nach 3 Tagen und Lymphozytenproliferation nach 6 Tagen in Kultur nachgewiesen. Die zytofluorimetrische Phänotypisierung der Zellen zeigte, daß am 6. Tage nahezu ausschließlich CD4 positive Lymphozyten (T-Helferzellen) proliferieren. Bei mikroskopischer Betrachtung der Zellkultur war eine Klonstruktur erkennbar.
Einzelheiten des Testergebnisses zeigt die Tafel 3.

Beispiel 4

Bei diesem Beispiel konnte bei einem Patienten eine latente Borrelien-Infektion durch das borrelienspezifische OspA-Antigen nachgewiesen werden.
Einzelheiten des Testergebnisses zeigt die Tafel 4.

Beispiel 5

Bei diesem Beispiel konnte eine zellvermittelte Hypersensitivitätsreaktion gegenüber Gold und Nickel nachgewiesen werden.
Am sechsten Tag konnte ein Anstieg der HLA-DR Expression auf CD4 und CD8 positiven Lymphozyten festgestellt werden. Der SI-Wert ist für Nickel und Gold größer 3. Es findet in Gegenwart von 2 bzw. 4 µg/ml Nickel 2 bzw. 4 µg/ml Gold eine deutliche IFN-γ-Sekretion in den Kulturüberstand statt, was für das Vorhandensein von Gold- und Nickel-spezifischen Effektorzellen spricht.

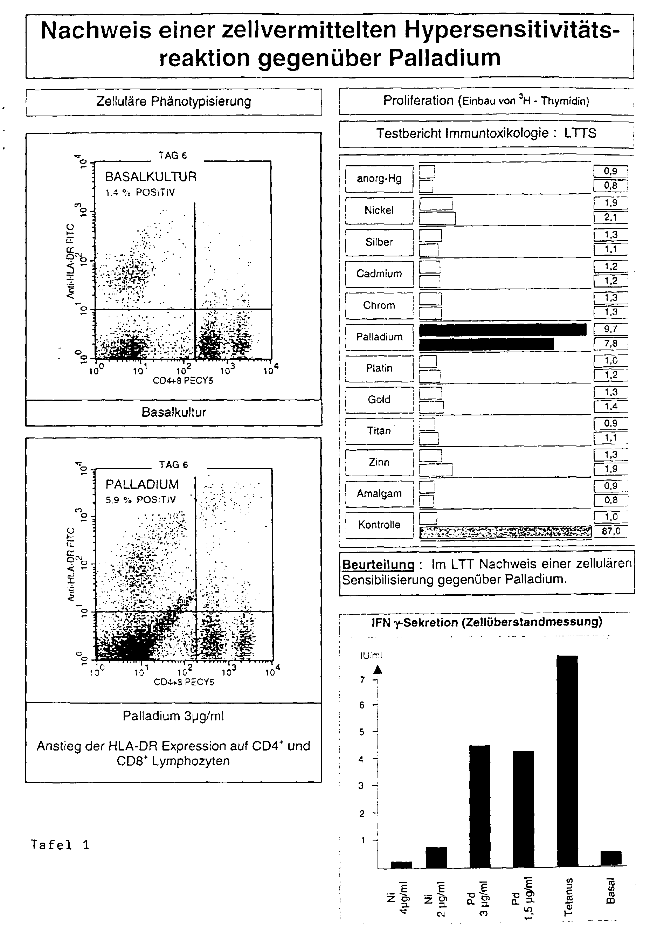

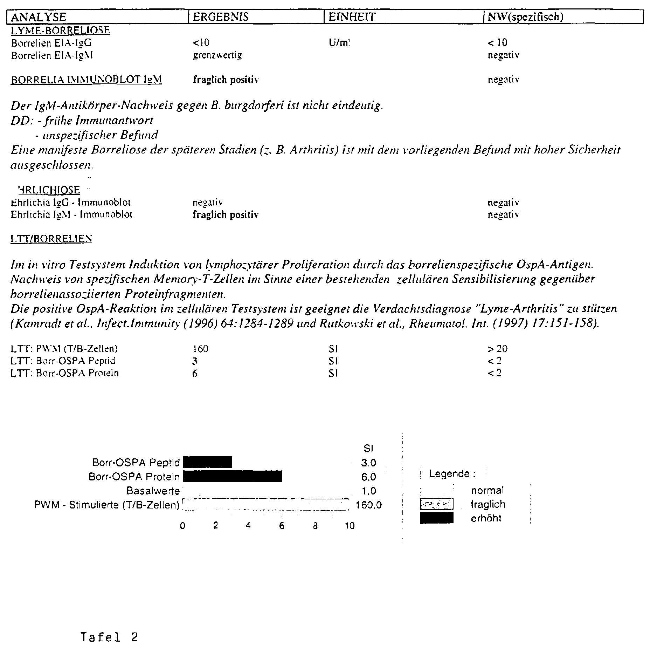

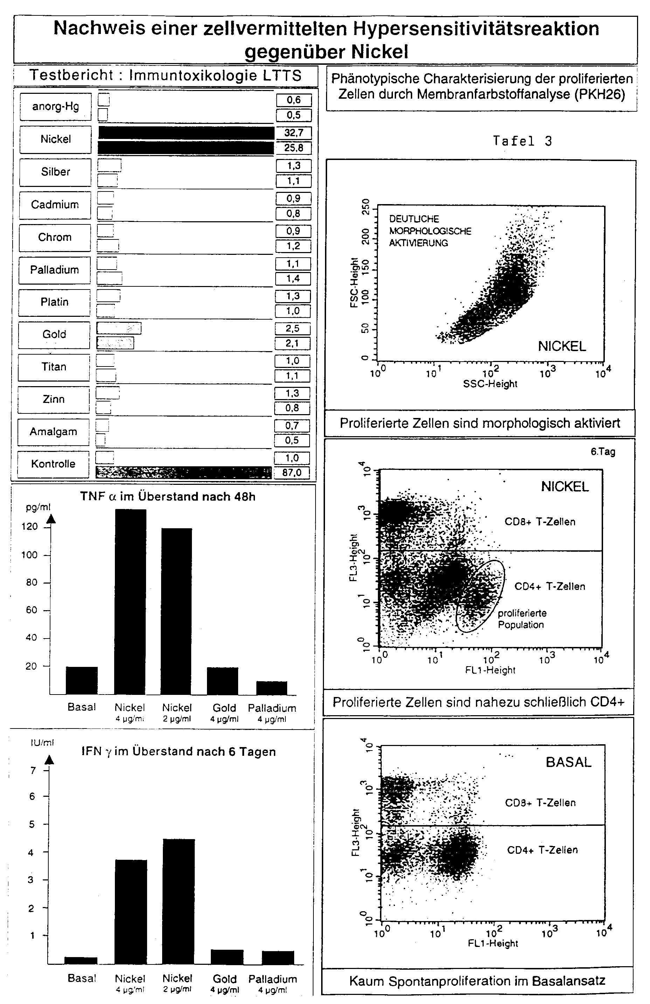

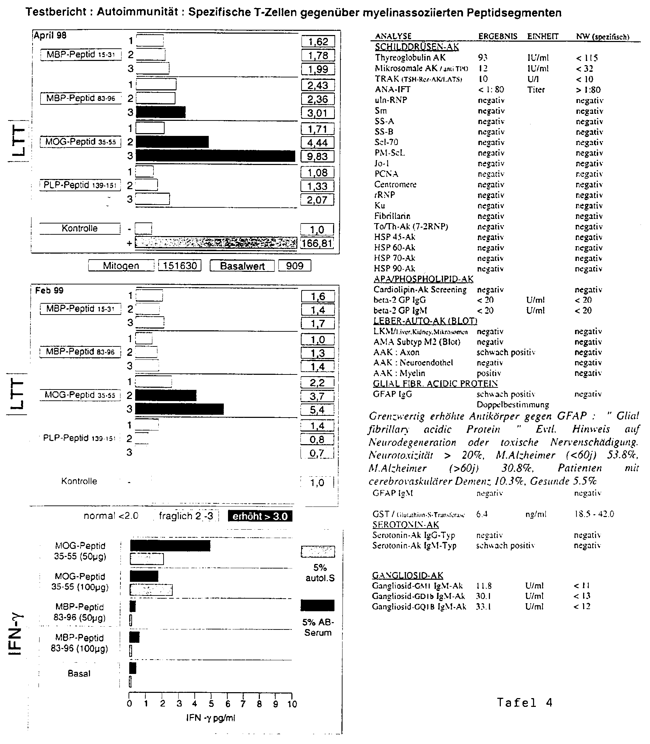

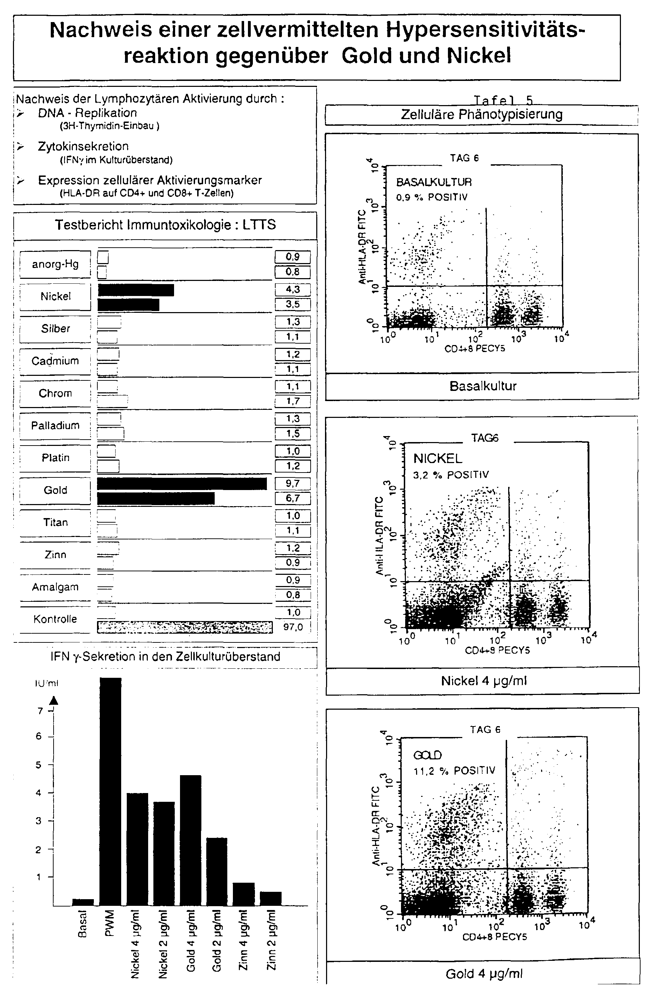

**Patentansprüche**

1. Verfahren zum in vitro-Nachweis von spezifisch antigen- bzw. allergenreaktiven Lymphozyten, bei dem man einer auf ein Antigen bzw. Allergen zu untersuchenden Zellkultur wenigstens eine Substanz zugibt, welche eine unspezifische Zell-Proliferation unterdrückt.

2. Verfahren nach Anspruch 1, dadurch gekennzeichnet, daß man die die unspezifische Zell-Proliferation unterdrückende(n) Substanz(en) aus der Familie der Human-Interferone auswählt.

3. Verfahren nach Anspruch 2, dadurch gekennzeichnet, daß man α-Interferon, insbesondere rekombinantes α-Interferon, zugibt.

4. Verfahren nach Anspruch 3, dadurch gekennzeichnet, daß man dem Test eine mononukleäre Zellfraktion eines Patienten zugrundelegt, welche Lymphozyten und Monozyten umfaßt.

5. Verfahren nach Anspruch 4, dadurch gekennzeichnet, daß man diese primäre Zellkultur auf eine Zellzahl von etwa 1,0 bis 1,5 x $10^6$ Zellen/ml einstellt.

6. Verfahren nach Anspruch 5, dadurch gekennzeichnet, daß man der so gewonnenen Zellsuspension die die unspezifische Zell-Proliferation unterdrückende Substanz, insbesondere humanes α-Interferon, in einer Menge zugibt, daß sie in der Zellkultur in einer Endkonzentration von etwa 100 bis 140 IU/ml vorliegt.

**7.** Verfahren nach Anspruch 6, dadurch gekennzeichnet, daß man diese Zellsuspenion, zusammen mit einem spezifischen Antigen bzw. Allergen unter in-vitro-Bedingungen inkubiert.

**8.** Verfahren nach Anspruch 7, dadurch gekennzeichnet, daß man nach beendeter Inkubationsphase entweder die Kulturüberstände zellfrei gewinnt und die darin enthaltenen sezernierten Zytokine in üblicher Weise bestimmt, oder die Zellsuspension mit einem radioaktiven DNS-Baustein inkubiert, die proliferierten Zellen danach erntet und zählt.

**9.** Verfahren nach einem der Ansprüche 1 bis 8, dadurch gekennzeichnet, daß man als Serumzusatz für die Inkubation vorzugsweise Serum des zu untersuchenden Patienten einsetzt.

**10.** Verfahren nach einem der Ansprüche 1 bis 9, dadurch gekennzeichnet, daß man den in vitro-Nachweis in einer vollautomatisierten Analyseapparatur durchführt.

**11.** Verfahren nach einem der Ansprüche 1 bis 9, dadurch gekennzeichnet, daß man den in vitro-Nachweis auf Mikrotiterplatten, insbesondere 96-well-Mikrotiterplatten, durchführt.

**12.** Verfahren nach Anspruch 11, dadurch gekennzeichnet, daß man vorab mit Antigen bzw. Allergen beladene Platten verwendet.

**13.** Verfahren nach einem der Ansprüche 8 bis 12, dadurch gekennzeichnet, daß man bei dem Nachweis pro zu testendem Antigen bzw. Allergen zur Ermittlung eines Mittelwertes drei Ansätze fährt und man gleichzeitig die Basalproliferation, d.h. ohne Zugabe von Antigen bzw. Allergen bestimmt, um die erhaltenen Ergebnisse damit ins Verhältnis zu setzen.

**14.** Verfahren nach Anspruch 13, dadurch gekennzeichnet, daß man den Stimulationsindex (SI) nach der Gleichung

$$SI = cpm\ in\ induziert\ proliferierender\ Kultur/cpm\ in\ basal\ proliferierender\ Kultur$$

berechnet.

**15.** Verfahren nach jedem der Ansprüche 1 bis 14 zum in-vitro-Nachweis einer spezifischen Sensibilisierung (Allergie).

**16.** Verfahren nach jedem der Ansprüche 1 bis 15 zum positiven Nachweis von spezifischen Metallsensibilisierungen.

**17.** Verfahren nach jedem der Ansprüche 1 bis 15 zum positiven Nachweis von autoreaktiven T-Zellen.

**18.** Verfahren nach jedem der Ansprüche 1 bis 15 zum positiven Nachweis von latenten Infektionen.

**19.** Nachweis-Kit zur Durchführung des Verfahrens nach einem der Ansprüche 1 bis 17, umfassend die mit Antigen bzw. Allergen und Kontrollsubstanzen beladenen Mikrotiterplatten.

# Nachweis einer zellvermittelten Hypersensitivitäts-reaktion gegenüber Palladium

## Zelluläre Phänotypisierung

TAG 6
BASALKULTUR
1.4 % POSITIV

Basalkultur

TAG 6
PALLADIUM
5.9 % POSITIV

Palladium 3µg/ml

Anstieg der HLA-DR Expression auf CD4⁺ und CD8⁺ Lymphozyten

Tafel 1

## Proliferation (Einbau von $^{3}$H - Thymidin)

Testbericht Immuntoxikologie : LTTS

| | | |
|---|---|---|
| anorg-Hg | | 0,9 |
| | | 0,8 |
| Nickel | | 1,9 |
| | | 2,1 |
| Silber | | 1,3 |
| | | 1,1 |
| Cadmium | | 1,2 |
| | | 1,2 |
| Chrom | | 1,3 |
| | | 1,3 |
| Palladium | | 9,7 |
| | | 7,8 |
| Platin | | 1,0 |
| | | 1,2 |
| Gold | | 1,3 |
| | | 1,4 |
| Titan | | 0,9 |
| | | 1,1 |
| Zinn | | 1,3 |
| | | 1,9 |
| Amalgam | | 0,9 |
| | | 0,8 |
| Kontrolle | | 1,0 |
| | | 87,0 |

**Beurteilung** : Im LTT Nachweis einer zellulären Sensibilisierung gegenüber Palladium.

### IFN γ-Sekretion (Zellüberstandmessung)

IU/ml

Ni 4µg/ml, Ni 2 µg/ml, Pd 3 µg/ml, Pd 1,5 µg/ml, Tetanus, Basal

| ANALYSE | ERGEBNIS | EINHEIT | NW(spezifisch) |
|---|---|---|---|
| LYME-BORRELIOSE | | | |
| Borrelien EIA-IgG | <10 | U/ml | < 10 |
| Borrelien EIA-IgM | grenzwertig | | negativ |
| | | | |
| BORRELIA IMMUNOBLOT IgM | fraglich positiv | | negativ |

*Der IgM-Antikörper-Nachweis gegen B. burgdorferi ist nicht eindeutig.*
*DD: - frühe Immunantwort*
*     - unspezifischer Befund*
*Eine manifeste Borreliose der späteren Stadien (z. B. Arthritis) ist mit dem vorliegenden Befund mit hoher Sicherheit ausgeschlossen.*

| HRLICHIOSE | | | |
|---|---|---|---|
| Ehrlichia IgG - Immunoblot | negativ | | negativ |
| Ehrlichia IgM - Immunoblot | fraglich positiv | | negativ |

LTT/BORRELIEN

*Im in vitro Testsystem Induktion von lymphozytärer Proliferation durch das borrelienspezifische OspA-Antigen.*
*Nachweis von spezifischen Memory-T-Zellen im Sinne einer bestehenden zellulären Sensibilisierung gegenüber borrelienassoziierten Proteinfragmenten.*
*Die positive OspA-Reaktion im zellulären Testsystem ist geeignet die Verdachtsdiagnose "Lyme-Arthritis" zu stützen (Kamradt et al., Infect.Immunity (1996) 64:1284-1289 und Rutkowski et al., Rheumatol. Int. (1997) 17:151-158).*

| LTT: PWM (T/B-Zellen) | 160 | SI | > 20 |
|---|---|---|---|
| LTT: Borr-OSPA Peptid | 3 | SI | < 2 |
| LTT: Borr-OSPA Protein | 6 | SI | < 2 |

Tafel 2

# Nachweis einer zellvermittelten Hypersensitivitätsreaktion gegenüber Nickel

## Testbericht : Immuntoxikologie LTTS

| | | |
|---|---|---|
| anorg-Hg | | 0,6 |
| | | 0,5 |
| Nickel | | 32,7 |
| | | 25,8 |
| Silber | | 1,3 |
| | | 1,1 |
| Cadmium | | 0,9 |
| | | 0,8 |
| Chrom | | 0,9 |
| | | 1,2 |
| Palladium | | 1,1 |
| | | 1,4 |
| Platin | | 1,3 |
| | | 1,0 |
| Gold | | 2,5 |
| | | 2,1 |
| Titan | | 1,0 |
| | | 1,1 |
| Zinn | | 1,3 |
| | | 0,8 |
| Amalgam | | 0,7 |
| | | 0,5 |
| Kontrolle | | 1,0 |
| | | 87,0 |

**TNF α im Überstand nach 48h**

pg/ml

Basal | Nickel 4 µg/m; | Nickel 2 µg/ml | Gold 4 µg/ml | Palladium 4 µg/ml

**IFN γ im Überstand nach 6 Tagen**

IU/ml

Basal | Nickel 4 µg/ml | Nickel 2 µg/ml | Gold 4 µg/ml | Palladium 4 µg/ml

## Phänotypische Charakterisierung der proliferierten Zellen durch Membranfarbstoffanalyse (PKH26)

Tafel 3

FSC-Height

DEUTLICHE MORPHOLOGISCHE AKTIVIERUNG

NICKEL

SSC-Height

**Proliferierte Zellen sind morphologisch aktiviert**

6.Tag

NICKEL

FL3-Height

CD8+ T-Zellen

CD4+ T-Zellen

proliferierte Population

FL1-Height

**Proliferierte Zellen sind nahezu schließlich CD4+**

BASAL

FL3-Height

CD3+ T-Zellen

CD4+ T-Zellen

FL1-Height

**Kaum Spontanproliferation im Basalansatz**

## Testbericht : Autoimmunität : Spezifische T-Zellen gegenüber myelinassoziierten Peptidsegmenten

**April 98** (LTT)

| MBP-Peptid 15-31 | 1 | 1,62 |
| | 2 | 1,78 |
| | 3 | 1,99 |
| MBP-Peptid 83-96 | 1 | 2,43 |
| | 2 | 2,36 |
| | 3 | 3,01 |
| MOG-Peptid 35-55 | 1 | 1,71 |
| | 2 | 4,44 |
| | 3 | 9,83 |
| PLP-Peptid 139-151 | 1 | 1,08 |
| | 2 | 1,33 |
| | 3 | 2,07 |
| Kontrolle | - | 1,0 |
| | + | 166,81 |

| Mitogen | 151630 | Basalwert | 909 |

**Feb 99** (LTT)

| MBP-Peptid 15-31 | 1 | 1,6 |
| | 2 | 1,4 |
| | 3 | 1,7 |
| MBP-Peptid 83-96 | 1 | 1,0 |
| | 2 | 1,3 |
| | 3 | 1,4 |
| MOG-Peptid 35-55 | 1 | 2,2 |
| | 2 | 3,7 |
| | 3 | 5,4 |
| PLP-Peptid 139-151 | 1 | 1,4 |
| | 2 | 0,8 |
| | 3 | 0,7 |
| Kontrolle | - | 1,0 |

normal <2.0    fraglich 2 -3    erhöht > 3.0

IFN-γ

MOG-Peptid 35-55 (50µg)
MOG-Peptid 35-55 (100µg)
MBP-Peptid 83-96 (50µg)
MBP-Peptid 83-96 (100µg)
Basal

5% autol.S
5% AB-Serum

0  1  2  3  4  5  6  7  8  9  10
IFN -γ pg/ml

| ANALYSE | ERGEBNIS | EINHEIT | NW (spezifisch) |
|---|---|---|---|
| **SCHILDDRÜSEN-AK** | | | |
| Thyreoglobulin AK | 93 | IU/ml | < 115 |
| Mikrosomale AK / anti TPO | 12 | IU/ml | < 32 |
| TRAK (TSH-Rez-AK/LATS) | 10 | U/l | < 10 |
| ANA-IFT | < 1: 80 | Titer | > 1:80 |
| uln-RNP | negativ | | negativ |
| Sm | negativ | | negativ |
| SS-A | negativ | | negativ |
| SS-B | negativ | | negativ |
| Scl-70 | negativ | | negativ |
| PM-Scl | negativ | | negativ |
| Jo-1 | negativ | | negativ |
| PCNA | negativ | | negativ |
| Centromere | negativ | | negativ |
| rRNP | negativ | | negativ |
| Ku | negativ | | negativ |
| Fibrillarin | negativ | | negativ |
| To/Th-Ak (7-2RNP) | negativ | | negativ |
| HSP 45-AK | negativ | | negativ |
| HSP 60-AK | negativ | | negativ |
| HSP 70-AK | negativ | | negativ |
| HSP 90-AK | negativ | | negativ |
| **APA/PHOSPHOLIPID-AK** | | | |
| Cardiolipin-Ak Screening | negativ | | negativ |
| beta-2 GP IgG | < 20 | U/ml | < 20 |
| beta-2 GP IgM | < 20 | U/ml | < 20 |
| **LEBER-AUTO-AK (BLOT)** | | | |
| LKM/Liver.Kidney.Mikrosomen | negativ | | negativ |
| AMA Subtyp M2 (Blot) | negativ | | negativ |
| AAK : Axon | schwach positiv | | negativ |
| AAK : Neuroendothel | negativ | | negativ |
| AAK : Myelin | positiv | | negativ |
| **GLIAL FIBR. ACIDIC PROTEIN** | | | |
| GFAP IgG | schwach positiv Doppelbestimmung | | negativ |

*Grenzwertig erhöhte Antikörper gegen GFAP : " Glial fibrillary acidic Protein " Evtl. Hinweis auf Neurodegeneration oder toxische Nervenschädigung. Neurotoxizität > 20%, M.Alzheimer (<60j) 53.8%, M.Alzheimer (>60j) 30.8%, Patienten mit cerebrovaskulärer Demenz 10.3%, Gesunde 5.5%*

| | | | |
|---|---|---|---|
| GFAP IgM | negativ | | negativ |
| GST / Glutathion-S-Transferase | 6.4 | ng/ml | 18.5 - 42.0 |
| **SEROTONIN-AK** | | | |
| Serotonin-Ak IgG-Typ | negativ | | negativ |
| Serotonin-Ak IgM-Typ | schwach positiv | | negativ |
| **GANGLIOSID-AK** | | | |
| Gangliosid-GM1 IgM-Ak | 11.8 | U/ml | < 11 |
| Gangliosid-GD1b IgM-Ak | 30.1 | U/ml | < 13 |
| Gangliosid-GQ1B IgM-Ak | 33.1 | U/ml | < 12 |

Tafel 4

# Nachweis einer zellvermittelten Hypersensitivitäts- reaktion gegenüber Gold und Nickel

Nachweis der Lymphozytären Aktivierung durch :
- DNA - Replikation
  (3H-Thymidin-Einbau )
- Zytokinsekretion
  (IFNγ im Kulturüberstand)
- Expression zellulärer Aktivierungsmarker
  (HLA-DR auf CD4+ und CD8+ T-Zellen)

## Testbericht Immuntoxikologie : LTTS

| | | |
|---|---|---|
| anorg-Hg | | 0,9 |
| | | 0,8 |
| Nickel | | 4,3 |
| | | 3,5 |
| Silber | | 1,3 |
| | | 1,1 |
| Cadmium | | 1,2 |
| | | 1,1 |
| Chrom | | 1,1 |
| | | 1,7 |
| Palladium | | 1,3 |
| | | 1,5 |
| Platin | | 1,0 |
| | | 1,2 |
| Gold | | 9,7 |
| | | 6,7 |
| Titan | | 1,0 |
| | | 1,1 |
| Zinn | | 1,2 |
| | | 0,9 |
| Amalgam | | 0,9 |
| | | 0,8 |
| Kontrolle | | 1,0 |
| | | 97,0 |

### IFN γ-Sekretion in den Zellkulturüberstand

IU/ml

Basal — PWM — Nickel 4 µg/ml — Nickel 2 µg/ml — Gold 4 µg/ml — Gold 2 µg/ml — Zinn 4 µg/ml — Zinn 2 µg/ml

## Tafel 5
### Zelluläre Phänotypisierung

TAG 6
BASALKULTUR
0,9 % POSITIV
Anti-HLA-DR FITC
CD4+8 PECY5

**Basalkultur**

TAG6
NICKEL
3,2 % POSITIV
Anti-HLA-DR FITC
CD4+8 PECY5

**Nickel 4 µg/ml**

TAG 6
GOLD
11,2 % POSITIV
Anti-HLA-DR FITC
CD4+8 PECY5

**Gold 4 µg/ml**